# EUROPEAN PATENT SPECIFICATION

(11) **EP 3 590 418 B1**
(45) Date of publication and mention of the grant of the patent: **25.06.2025**
(21) Application number: 18182302.2
(22) Date of filing: 06.07.2018
(51) Int. Cl.: A61B 5/00, A61B 5/113, A61B 5/0205

(54) **APPARATUS FOR MONITORING A PATIENT DURING HIS SLEEP**
VORRICHTUNG ZUR ÜBERWACHUNG EINES PATIENTEN WÄHREND SEINES SCHLAFES
APPAREIL DE SURVEILLANCE D'UN PATIENT PENDANT SON SOMMEIL

(43) Date of publication of application: 08.01.2020
(73) Proprietor: Yazigi, Raja, 1544 Gletterens (CH); Koller, Philippe, 2000 Neuchâtel (CH)
(72) Inventor: Yazigi, Raja, 1544 Gletterens (CH); Koller, Philippe, 2000 Neuchâtel (CH)
(74) Representative: P&TS SA (AG, Ltd.)

(56) References cited:
- WO-A1-2018/033889
- US-A1- 2013 030 257
- US-A1- 2015 173 672

## Description

### Field of the invention

The present invention concerns an apparatus for monitoring a patient during his sleep.

### Description of related art

Sleep apnea syndrome (SAS) is a sleep disorder in which an individual's breathing repeatedly stops and starts during sleep, sometimes tens or hundreds of times. One common form of SAS is the obstructive sleep apnea (OSA) where the pause in breathing is due to a blocked airway, usually when the soft tissue in the back of the throat collapses during sleep. Another form of SAS is central sleep apnea (CSA) where the pause is commanded from the brain.

If untreated, SAS can result in a number of health problems, including heart failure such as sudden cardiac death. Sudden death is widespread both among newborns and among adults.

Devices for monitoring the quality of the sleep during a so-called polysomnogram study are already known. Those devices electronically measure and record specific parameters of a patient during his sleep. The recordings are analyzed by a sleep specialist to determine whether or not the patient has sleep apnea. Those devices are usually intended for use in a hospital or sleep center for diagnostic purpose. Treatment of a diagnosed SAS includes for example changing habits (losing weight, reducing alcohol consumption) or surgical intervention.

US9202008 describes a device for monitoring breath with two belts, one for the abdominal breath and one for the thoracic breath. The monitoring can be used for diagnosis but not for taking immediate action in case of risk of sudden cardiac death.

EP1858410B1 discloses a reusable inductance plethysmograph transducer used for respiratory monitoring comprising 2 inductive sensors carried on 2 belts, one around the abdomen and the other around the chest. Both signals are monitored and interpreted by a polysomnographic technologist studying the patient.

WO2018033889 A1 discloses a portable device comprising a breathing sensor with two respiratory inductance plethysmography belts placed on the chest and abdomen and ECG electrodes monitoring the heart rate.

It is also known in the prior art that a baby can be easily saved if he is wakened up immediately after he stopped breathing during his sleep.

US2008183095 describes a breathing monitor for monitoring respiration, such as for detecting apnea events and/or preventing Sudden Infant Death. Visible and/or audible alarms are coupled to the system, and activated in the event that breathing stops for a predetermined period of time. Immediately wakening the child - or his parent - in case of breath pause reduces the risk of sudden death. However, many children and adults have multiple events of sleep apnea during each sleep; generating an alarm that wakes the child or his parent at each occurrence is neither acceptable nor even useful.

US2008183095 discloses an infant monitoring system comprising inductance-based sensors placed on the chest and abdomen of a child (e.g. integrated into garments such as infant sleepwear). A correlation between both signals is not mentioned.

### Brief summary of the invention

It is therefore an aim of the invention to propose a new device for monitoring sleep and reducing the risk of consequent pathologies.

According to the invention, these aims are achieved by means of a method for monitoring a patient during his sleep, comprises:
monitoring the thoracic breath of the patient;
monitoring the abdominal breath of the patient;
detecting a sleep apnea syndrome (SAS) event;
in reaction to the detection of N such SAS events, N being an integer equal or larger than 1, performing an additional measure to detect an abnormal condition.

The additional measure may be used for determining if the situation is critical and if an alarm or recording should be performed.

The output of said additional measure may be recorded for later analysis or transmission to a remote device.

An alarm may be sent in reaction to the detection of an abnormal condition.

The additional measure is only performed after N SAS events. Therefore, it only requires power when needed.

The monitoring of the thoracic breath and/or the monitoring of the abdominal breath may include a measure of the amplitude of a breath signal.

The monitoring of the thoracic breath and/or the monitoring of the abdominal breath may include a measure of the frequency of a breath signal.

The monitoring of the thoracic breath and/or the monitoring of the abdominal breath may include a measure of the phase of a breath signal, and/or a measure of the phase difference between the thoracic breath and the abdominal breath.

The additional measure performed in case of detection of SAS events may comprise an additional heart measure.

The additional heart measure may include a detection of an unexpected increase or decrease in the heart rate.

The additional heart measure may include a detection of arrhythmia.

The additional heart measure may include an electrocardiogram.

The additional heart measure may include a detection of the sleep quality.

The additional heart measure may include an evaluation of the oxygenation of the blood.

The additional measure may include an encephalogram.

The additional measure may include an evaluation of the oxygenation of the brain.

The detection of N SAS events may comprise a detection of an obstructive sleep apnea (OSA) event, the detection being based on the phase of the thoracic breath and on the phase of the abdominal breath.

The detection of N SAS events may comprise a detection of a hypopnea event.

The detection of N SAS events may include a detection of only such events longer than a predetermined threshold.

The duration threshold be dependent on the patient or on the age of the patient.

The additional measure may comprise a measure of the body position after said event. An abnormal condition, or at least an increased risk may be detected if the patient is sleeping on his stomach.

The additional measure may comprise a measure of body movements after said event. Fast movements after N SAS event may trigger an alarm.

The alarm may be a visual or audio alarm emitted by the patient-worn device, in order to wake the patient.

The alarm may be sent over a radiofrequency short distance interface to a proprietary, dedicated base station, for example a device within the same room as the patient.

A dedicated, proprietary base station is easier to certify for medical use than a smartphone or other general-purpose user equipment.

The alarm may be sent from the base station to a remote server, for example a server in the Internet. It can be forwarded to or retrieved from the server to a user equipment.

The detection of N SAS events and the decision to perform an additional measure are taken in a wearable device. A stand-alone device is easier to certify for medical use than a separate device; moreover, this reduces the risk of an alarm not being generated if a device is not powered.

An inertial sensor and an additional sensor of a different type may be used for monitoring said thoracic breath or said abdominal breath.

The thoracic breath and/or said abdominal breath may be measured with a first sensor, said measure may be corrected with a second sensor, for example in order to compensate for artifacts caused by body movements.

One of the two sensors may be an inertial sensor.

One of the two sensors may be an inductive sensor.

The number (N) of SAS event, and/or the minimal duration of a breathing pause in a SAS event, and/or thresholds used for evaluating the results of said additional measure, may be adapted to the age of the patient.

The user device may be personalized by entering the age of the patient.

The invention relates to a wearable device as defined in claim 1, comprising inter alia:
a first sensor for monitoring the thoracic breath of the patient;
a second sensor for monitoring the abdominal breath of the patient;
an embedded computing unit arranged for receiving signals output by said first sensor and by said second sensor, for detecting sleep apnea syndrome (SAS) events based on said signals, and for triggering an additional measure in reaction to the detection of N such SAS events, N being an integer equal or larger than 1.

The device may include a short distance wireless interface, said computing unit being arranged for evaluating said additional measure and for sending an alarm signal in reaction to the detection of an abnormal condition.

The device may further include a memory, said processing unit being arranged for recording the output of said additional measure for a later analysis.

The device may be arranged for detecting a sudden increase or decrease in the heart rate.

The device may be arranged for detecting arrhythmia.

The device may include an electrocardiogram sensor.

The device may comprise a sensor for evaluating the oxygenation of the blood.

The device may comprise a sensor for performing an encephalogram test.

The device may comprise an accelerometer for detecting body movements after said event.

The device may comprise a radiofrequency short distance interface for communicating with a proprietary, dedicated base station.

The device may comprise an inertial sensor and an additional sensor of a different type for monitoring said thoracic breath or for monitoring said abdominal breath.

The breath sensor may also be used for detecting whether the patient is awake or sleeping, based on the breathing frequency or amplitude; this information may be used for adapting a breathing amplitude threshold under which a SAS event is detected.

The device may be arranged for measuring said thoracic breath and/or said abdominal breath with one sensor and to correct it with a second sensor.

The device may comprise a memory for storing the age of said patient, said computing unit being arranged for adapting said number (N) of SAS events, and/or the minimal duration of a breathing pause in a SAS event, and/or thresholds used for evaluating the results of said additional measure, to the age of the patient.

### Brief Description of the Drawings

The invention will be better understood with the aid of the description of an embodiment given by way of example and illustrated by the figures, in which:
Fig. 1 is a schematic view of a system comprising an apparatus according to the invention.
Fig. 2 is a flowchart of a method using the apparatus according to the invention.

### Detailed Description of possible embodiments of the Invention

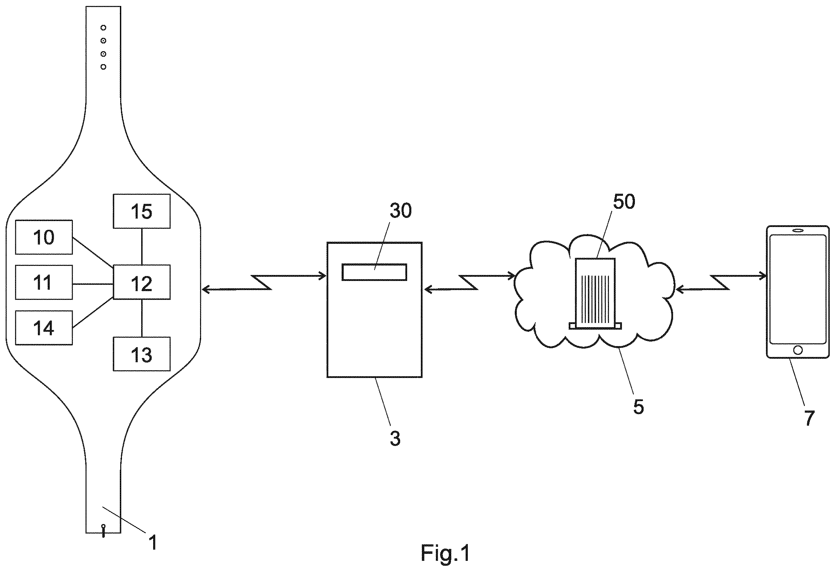

Figure 1 illustrates an example of an embodiment of a system according to the invention. It comprises a wearable device 1, such as a belt, a garment, a set of belts, etc. The device 1 comprises electronic components powered by a battery (not shown). It comprises notably a first sensor 10 for monitoring the thoracic breath of the patient during his sleep, and a second sensor 11 for monitoring the abdominal breath of the patient. The first and/or the second sensor may each be based on an inertial sensor for measuring the breath induced displacement of the skin, on inductive measure, capacitive measure, or any other measurement system suitable for measuring thoracic respectively abdominal breath.

The device 1 may include at least one additional sensor (not shown) for correcting or improving the measure made by either sensor 10 and/or sensor 11. For example, the device 1 may include an additional inertial sensor, such as an accelerometer or gyroscope, for detecting when the patient moves and compensating artefacts caused by this movement on the signal output by sensors 10 or 11.

The device 1 is placed on the patient's body during his sleep with the sensors 10, 11 placed around his chest and abdomen respectively, to measure his breathing.

A computing unit 12, such as a microprocessor or microcontroller, is embedded in the device 1 and receives breathing signals output by the first sensor and by the second sensor. The computing unit 12 analyses those signals and detects sleep apnea events.

The detection of sleep apnea events is based on the amplitude of the breathing signals output by the sensors 10, 11, on their frequency, on their phase, on their difference of phase, or on any combination thereof. For example, a sleep apnea event may be detected when the amplitude of the breathing signals is nil, or below an amplitude threshold, during a duration greater than a threshold. An obstructive sleep apnea (OSA) event may also be detected when the thoracic and abdominal breathing signals are out of phase (as the chest expands with inspiration, the abdomen contracts) during a duration greater than a threshold. A sleep apnea event may also be detected when the frequency of at least one breathing signal is under a frequency threshold. A hypopnea event may also be detected.

The amplitude threshold, the duration thresholds, and/or the frequency threshold may be predetermined. Alternatively, at least one of those thresholds may depend on the patient, and for example be adapted to the patient, or computed depending on his age, or dependent on his antecedents or medical history, or on whether the patient is sleeping or not.

The computing unit 12 includes a counter, for example a software counter (not shown) for counting the number of SAS events detected during each night or each period of sleep. As will be described later, an additional measure, using for example an additional sensor, is triggered by the computing unit 12 when the number of detected SAS events during one sleep period exceeds a threshold N. The threshold N may depend on the patient and/or on his age. In one embodiment, the counter is periodically reset, for example every hour. In one embodiment, the threshold N is set at at least 15 SAS per hour, or at at least 30 SAS per hour.

In one embodiment, the severity of each SAS event, for example its duration, or other parameters of the breathing signals and/or of other signals, is also considered for determining whether an additional measure should be performed. For example, a SAS event with a duration exceeding a third duration threshold may trigger an immediate additional measure, even if this is the first SAS event in the sleep period.

The purpose of the additional measure is to determine the seriousness of the situation (i.e. the risk of sudden cardiac death) and whether an alarm should be triggered, or at least whether additional physiological parameters should be recorded for later analysis.

The additional measure is preferably only triggered after detection of N SAS events during a period or after a serious SAS event. Therefore, this measure does not disturb the patient's sleep when not needed, and does not drain the battery of the user-worn device more than necessary.

The additional measure may comprise a heart measure, for example a measure of the heart rate. If the number of beats per minute exceeds a high bpm threshold, for example 75 bpm or more, or is under a low bpm threshold, for example 25 bpm or less, an alarm is generated. Alternatively, the additional measure comprises a detection of a change of heart rate, for example an increase or a decrease of more than 20%, or more than 25%. The bpm threshold may be adapted to the patient age, condition, usual threshold, and/or efforts during his sleep.

The additional measure may include a detection of arrhythmia, i.e., irregularities in the heart rhythm.

The additional measure may comprise an electrocardiogram, using electrodes placed on the patient's skin.

The additional measure may comprise a determination of the quality of sleep, based for example on detection of episodes of sleep, type of sleep, based for example on signals output by any of the sensors 10, 11, 14. Alternatively, the additional measure may be based on an electro-oculogram to record eye movements and derive the type of sleep during each phase.

The additional measure may comprise an evaluation of the oxygenation of the blood, using for example an oximeter probe on a patient's finger to measure the amount of oxygen in his blood.

The additional measure may comprise an encephalogram to measure, or a measure of the oxygenation of the brain.

The additional measure may use a snore microphone to record and evaluate snoring activity.

The additional measure may comprise a determination of the body position after the N events; for example, an alarm may be detected if the patient is sleeping on his stomach, while the situation will be considered less serious if he is sleeping on his back.

The additional measure may comprise a detection of body movements after N events, for example detection of unusual or rapid movements. This detection may use an accelerometer or gyroscope on the patient's body.

The additional measure may comprise an electromyogram to record muscle activity such as teeth grinding, leg movements, etc...

The computing unit 12 receives an output from the additional sensor 14 in response to such a request for an additional measure, and decides on an action to be taken based on this output and possibly on the previous outputs from sensors 10,11.

In one option, the computing unit triggers an acoustic, visual and/or tactile alarm if the output of the additional measure indicates a serious condition when it is safer to wake the patient. The alarm may be emitted by an alarm unit 15 in the device 1, such as a loudspeaker, a buzzer, a lamp, a vibrating unit, etc., on the device 1 itself.

Alternatively, when such a serious condition is detected, an alarm signal is sent over a wireless interface 13 to a base station 3 at short distance, for example in the same room. The alarm may be sent for example over a Bluetooth smart interface, a ZigBee interface, or a proprietary interface. "short distance" means in this context a few meters, for example less than 20 meters, preferably less than 10 meters.

The base station 3 is preferably a dedicated, proprietary device, rather than a smart-phone, personal computer or other generic purpose equipment. The use of a dedicated proprietary device allows for an easier medical certification of the system.

The base station 3 may be plugged into a power socket. It may comprise an alarm unit 30 to emit an audible or visual signal when a serious condition has been detected.

The base station may be connected to a remote server 5, for example a server in a cloud 5. The connexion between the base station 3 and the remote server is preferably a long range, low debit data connection, for example a LoRa connection. The Server 50 may store measuring parameters of the patient, and make those parameters and analysis outputs available to the patient or to a doctor over a user terminal 7 connected to the server 50 over the Internet.

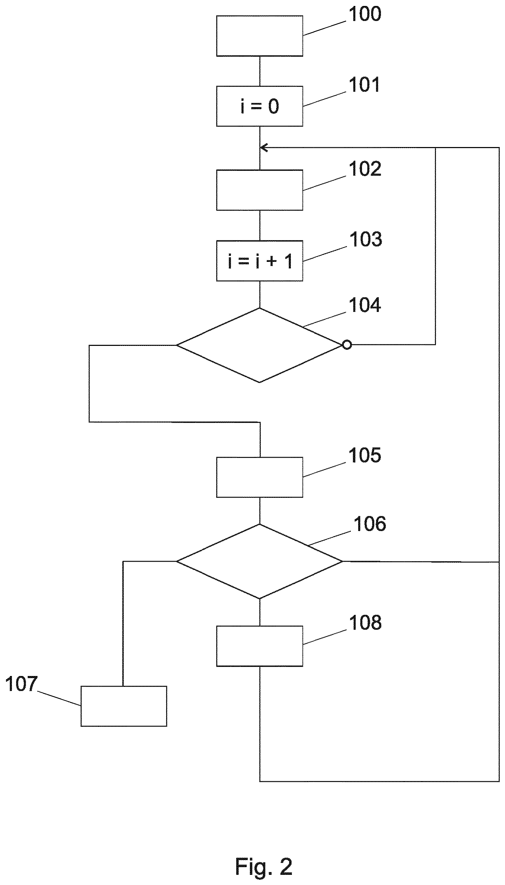

Figure 2 illustrates an example of flow chart of the method. The method starts at step 100 when the device which is installed on the patient is switched on or reset. At step 101, an increment value i is reset (i=0).

At step 102, the device 1 measures the abdomen and thoracic breathing with sensors 10, 11, and detects sleep apnea syndrome (SAS) events, as previously indicated. The amplitude of breath at which a SAS is detected may depend on the patient age, history, and on his sleeping statute (i.e. one expects a low amplitude of breath when the patient sleeps).

At step 103, when a SAS has been detected, the increment value is incremented.

At step 104, a test is made to verify if the number i of SAS exceeds a number threshold N, or if the seriousness of the last event exceeds a seriousness event. The threshold may depend on the patient, or on his age.

If not (for example at the first SAS event in a sleep period), the program returns to step 102.

If the threshold number of SAS has been reached, or if the last SAS is serious, the program goes to step 105, where an additional measure is made using the additional sensor 14, as previously described.

At step 106, the output of this additional measure is evaluated, possibly in combination with the previous outputs from the sensors 10, 11. This evaluation is performed by the embedded processing unit 12, and is therefore not dependent on the availability of the wireless connection with the base station 3.

If the additional measure determines that the condition is abnormal, notably when the risk of sudden death is high, an alarm is emitted at step 107 by the alarm unit 15, and/or an alarm signal is sent to the base station 3 and emitted by the alarm unit 30.

If the additional measure determines that the condition is serious or interesting, but does not require the patient to be waken, the output of the sensor 14 is stored locally in the device 1 during step 108, and/or transmitted to the base station and possibly to the remote server for later analysis and diagnosis. The process then returns to step 102.

If the additional measure determines that there is no risk, for example in the case of a false alarm when the sensors 10 and/or 11 are not functioning or badly connected, the program returns to step 102.

The counter may be periodically reset, for example every hour or every 30 minutes, to detect a number of SAS events in each period, for example the number of SAS per hour.

## Claims

1. A wearable device (1) comprising:
a first sensor (10) for monitoring the thoracic breath of the patient;
a second sensor (11) for monitoring the abdominal breath of the patient;
an embedded computing unit (12) configured for receiving signals output by said first sensor and by said second sensor, said embedded computing unit further configured for detecting sleep apnea syndrome (SAS) events based on said signals, **characterised in** said embedded computing unit comprising a counter adapted for counting the number of SAS event detected during each night or period of sleep, said embedded computing unit being further arranged for triggering an additional measure if the number of detected SAS events exceeds a threshold N or after a serious SAS event, corresponding to a SAS event exceeding a duration threshold, said additional measure being triggered by the computing unit only after said number N of SAS is detected during a period or after a SAS event exceeds said duration threshold.

2. The device of claim 1, comprising a short distance wireless interface, said computing unit being configured for evaluating said additional measure and for sending an alarm signal in reaction to the detection of an abnormal condition.

3. The device of one of the claims 1 and 2, comprising a heart signal detector for performing a heart measurement as additional measure.

## Patentansprüche

1. Ein tragbares Gerät (1), umfassend:
- einen ersten Sensor (10) zum Überwachen der Brustatmung des Patienten;
- einen zweiten Sensor (11) zum Überwachen der Bauchatmung des Patienten;
- eine eingebettete Recheneinheit (12), die zum Empfangen von Signalen konfiguriert ist, die von dem ersten Sensor und dem zweiten Sensor ausgegeben werden, wobei die eingebettete Recheneinheit weiter zum Erkennen von Schlafapnoe-Syndrom (SAS) Ereignissen auf der Grundlage der genannten Signale konfiguriert ist, **dadurch gekennzeichnet, dass** die genannte eingebettete Recheneinheit einen Zähler umfasst, der die Anzahl der während jeder Nacht oder Schlafperiode erkannten SAS-Ereignissen zählt, wobei die eingebettete Recheneinheit weiter dazu ausgerichtet ist, eine zusätzliche Maßnahme auszulösen, wenn die Anzahl der erfassten SAS-Ereignissen einen Schwellenwert N überschreitet, oder nach einem schwerwiegenden SAS-Ereignis, das einem SAS-Ereignis entspricht, das einen Dauer-Schwellenwert überschreitet, wobei die genannte zusätzliche Maßnahme von der Recheneinheit erst ausgelöst wird, nachdem die Anzahl N von SAS während einer Periode erfasst ist, oder nachdem ein SAS-Ereignis den Dauer-Schwellenwert überschritten hat.

2. Vorrichtung nach Anspruch 1, umfassend eine drahtlose Nahbereichsschnittstelle, wobei die Recheneinheit dazu konfiguriert ist, die zusätzliche Maßnahme auszuwerten und als Reaktion auf die Erkennung eines abnormalen Zustands ein Alarmsignal zu senden.

3. Vorrichtung nach einem der Ansprüche 1 oder 2, umfassend einen Herzsignaldetektor zur Durchführung einer Herzmessung als zusätzliche Maßnahme.

## Revendications

1. Dispositif portable (1) comprenant :
- un premier capteur (10) pour surveiller la respiration thoracique du patient;
- un deuxième capteur (11) pour surveiller la respiration abdominale du patient ;
- une unité de calcul intégrée (12) configurée pour recevoir des signaux émis par ledit premier capteur et par ledit deuxième capteur, ladite unité de calcul intégrée étant en outre configurée pour détecter des événements du syndrome d'apnée du sommeil (SAS) sur la base desdits signaux, **caractérisé en ce que** ladite unité de calcul intégrée comprend un compteur adapté pour compter le nombre d'événements SAS détectés pendant chaque nuit ou période de sommeil, ladite unité de calcul intégrée étant en outre agencée pour déclencher une mesure supplémentaire si le nombre d'événements SAS détectés dépasse un seuil N ou après un événement SAS grave, correspondant à un événement SAS dépassant un seuil de durée, ladite mesure supplémentaire étant déclenchée par l'unité de calcul uniquement après que ledit nombre N de SAS a été détecté pendant une période ou après qu'un événement SAS a dépassé ledit seuil de durée.

2. Le dispositif selon la revendication 1, comprenant une interface sans fil à courte distance, ladite unité de calcul étant configurée pour évaluer ladite mesure supplémentaire et pour envoyer un signal d'alarme en réaction à la détection d'une condition anormale.

3. Le dispositif selon l'une des revendications 1 et 2, comprenant un détecteur de signal cardiaque pour effectuer une mesure cardiaque comme mesure supplémentaire.
